(19) Europäisches Patentamt / European Patent Office / Office européen des brevets

(11) **EP 2 578 164 A1**

(12) **EUROPEAN PATENT APPLICATION**

(43) Date of publication:
**10.04.2013 Bulletin 2013/15**

(51) Int Cl.:
*A61B 8/08* *(2006.01)*          *A61B 5/00* *(2006.01)*

(21) Application number: **12185176.0**

(22) Date of filing: **20.09.2012**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority: **04.10.2011 JP 2011219879**

(71) Applicant: **Canon Kabushiki Kaisha
Tokyo 146-8501 (JP)**

(72) Inventor: **Miyasato, Takuro
Tokyo, Tokyo 146-8501 (JP)**

(74) Representative: **TBK
Bavariaring 4-6
80336 München (DE)**

(54) **Acoustic wave acquiring apparatus**

(57) The present invention provides an acoustic wave acquiring apparatus including: a holding plate configured to hold an object via acoustic matching agent; an acoustic wave detector configured to receive an acoustic wave generated from the object irradiated with light from a light source and convert the acoustic wave to an electric signal; and a processor configured to generate characteristic information of the inside of the object by using the electric signal. In the acoustic wave acquiring apparatus, the holding plate is subjected to hydrophilic process or the acoustic matching agent contains surfactant.

FIG. 2

**Description**

BACKGROUND OF THE INVENTION

Field of the Invention

The present invention relates to an acoustic wave acquiring apparatus.

Description of the Related Art

An optical imaging apparatus for irradiating an object with light from a light source such as a laser and obtaining space distribution information on optical characteristics in the object from the light propagated in the object is positively studied mainly in the medical field using a living body as the object. As one of such optical imaging techniques, photoacoustic tomography (hereinbelow, PAT) is disclosed in U.S. Patent No. 5,713,356 (Patent Literature 1 : PTL 1).

In the PAT, an acoustic wave (ultrasound wave) is generated when a light, which is emitted from a light source such as a nanosecond pulse laser, is absorbed by a living tissue via propagation and diffusion in a living body (object), and a probe detects the acoustic wave. By analyzing the detected acoustic wave, information related to the optical characteristic in the object is visualized. Concretely, an optical energy absorption density distribution and an optical absorption coefficient distribution computed from the optical energy absorption density distribution are calculated as the optical characteristics in the object based on the initial sound pressure distribution of the acoustic wave obtained from the object.

In the case of measuring a breast or other tissues of a human by the PAT, there is a case such that the breast is sandwiched by two holding plates and fixed, as described in U.S. Patent No. 5,713,356 and Srirang Manohar, et al., "Characterization of a clinical prototype for photoacoustic mammography and some phantom studies", Proc. of SPIE, p27 (Non Patent Literature 1 : NPL 1). At this time, the breast is irradiated with light which passed through the holding plates and a photoacoustic wave generated in the breast is detected via the holding plates. As the material of the holding plate, acrylic or polycarbonate can be used in consideration of light permeability. When air enters between the breast and the holding plate, the acoustic wave from the inside of the living body cannot be detected or it becomes difficult to detect it. It is consequently necessary to thinly apply acoustic matching agent to the entire contact face between the breast and the holding plate. As the acoustic matching agent, liquid such as water or jel used in an ultrasonograph can be used.

In the case of irradiating a human body with laser light, the MPE (Maximum Permissible Exposure) on the skin is determined by the JIS standards (C6802 and IEC60825-1). Therefore, an apparatus has to be designed so that intensity of laser light emitted to the surface of a human body does not exceed the MPE.

PTL 1 : U.S. Patent No. 5,713,356

NPL 1 : Srirang Manohar, et al., Characterization of a clinical prototype for photoacoustic mammographu and some phantom studies, Proc. Of SPIE, p27

SUMMARY OF THE INVENTION

In the case of irradiating an object over a holding plate with light and performing measurement by the PAT, it is expected that a drop of acoustic matching agent (water or jel) is adhered to a holding plate through which light passes. In the case of using acrylic or polycarbonate as the material of the holding plate in consideration of permeability of light, the acoustic matching agent is adhered to the holding plate at a large contact angle, so that the acoustic matching agent becomes a lens having high light focusing degree. Hereinafter, water, liquid, jel, or the like for obtaining an acoustic contact between the breast and the holding plate will be generically called as the acoustic matching agent.

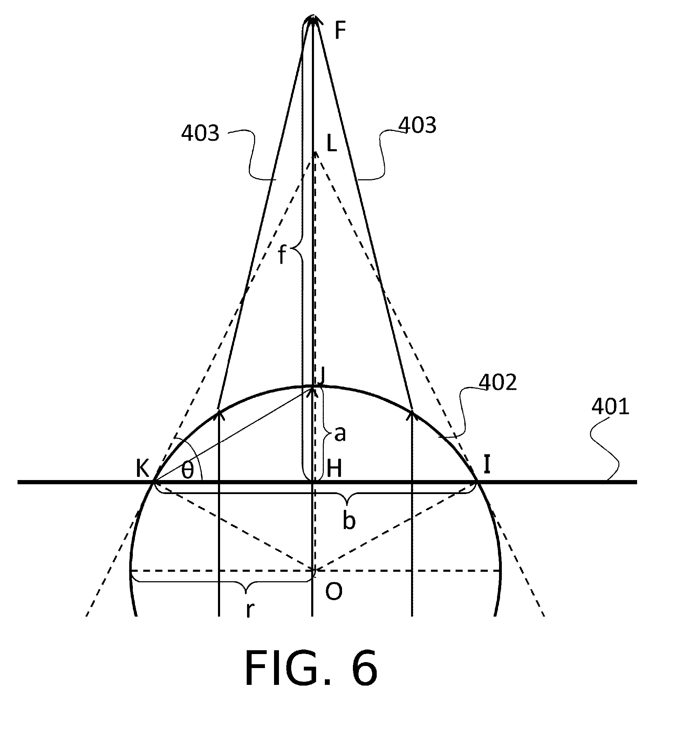

Generally, the shape of the waterdrop on a solid is simply described as a part of a circle as shown in FIG. 6. In FIG. 6, a straight line 401 is regarded as a solid surface, a circle is drawn around point O as a center, and the circle is divided in two parts by the solid surface 401. A part surrounded by an ark IJK on the upper side of the solid surface and a straight line KHI is a droplet 402. FIG. 6 is a cross section of the waterdrop of the liquid seen from a side. The cross point of an extension line passing the center O of the circle and the center H of a line KI and, tangents of the circle at points K and I, is a point L. In the diagram, when the diameter of the waterdrop is "b" and the height of the waterdrop is "a", the contact angle $\theta$ is approximately calculated by the equation (1) from the proposition and similarity of the internal angle of the triangle. The height of the waterdrop is height in the center of the circle.

$$\theta = 2 \cdot \tan^{-1}(2a/b) \quad \dots \ (1)$$

The diameter of the waterdrop refers to the diameter of the circle of a region in which the waterdrop and a solid are in contact.

FIG. 6 also shows a light 403 passed through the waterdrop. The light 403 is focused at the point F on the extension line of the straight line OL. When the curvature radius of the waterdrop is "r" and the refractive index for air of water is "n", the distance "f" of HF is calculated by the equation (2).

$$f \approx r/(n-1) \quad \dots \ (2)$$

Further, when the equation (2) is converted according to FIG. 6, the focal distance "f" is expressed by the equation (3).

$$f \approx b/(2 \cdot (n-1) \cdot \sin\theta) \dots \ (3)$$

It is understood from the equations (1) to (3) that, in the case where the drop of acoustic matching agent becomes a lens having high focusing degree, light passed through the acoustic matching agent is focused in the focal position, so that the optical irradiation density on the surface of the object in a position apart from the holding plate becomes locally high.

Consequently, even in a position where the optical irradiation density distribution becomes locally high by the lens effect, the average density of light emitted to the surface of the object has to be set lower than that in the MPE so as not to exceed the MPE of the JIS standard. As a result, the invasion length of light in the object becomes short and the intensity of the acoustic wave generated becomes low. It causes a problem such that an image of a deep part of the object cannot be constructed well.

The present invention has been achieved in consideration of the above problem. The present invention provides an apparatus for excellently constructing an image of a deep part of an object by increasing average density of irradiation light in photoacoustic measurement using holding plates and acoustic matching agent.

The present invention in its first aspect provides an acoustic wave acquiring apparatus as specified in claims 1 and 2.

The present invention in its second aspect provides an acoustic wave acquiring apparatus as specified in claims 3 and 4.

According to the present invention, an apparatus for excellently forming an image of a deep part of an object by increasing average density of irradiation light in photoacoustic measurement using holding plates and acoustic matching agent can be provided.

Further features of the present invention will become apparent from the following description of exemplary embodiments with reference to the attached drawings.

BRIEF DESCRIPTION OF THE DRAWINGS

FIG. 1 is a diagram showing a basic configuration of a photoacoustic imaging apparatus;
FIG. 2 is a diagram showing the configuration of a photoacoustic imaging apparatus according to a first embodiment;
FIG. 3 is a diagram showing the configuration of a photoacoustic imaging apparatus according to a second embodiment;
FIG. 4 is a flowchart showing the procedure of measurement according to the first embodiment;
FIG. 5 is a flowchart showing the procedure of measurement according to the second embodiment;
FIG. 6 is a diagram showing light focusing by a waterdrop on a holding plate; and
FIG. 7 is a diagram showing focusing of diffusion light by a waterdrop on a holding plate.

DESCRIPTION OF THE EMBODIMENTS

Hereinafter, preferred embodiments of the present invention will be described with reference to the drawings. The scope of the invention is not limited to the embodiments. An acoustic wave acquiring apparatus of the present invention receives an acoustic wave generated by an optical absorber in an object by photoacoustic effect when the object is irradiated with light (electromagnetic wave) by an element of an acoustic wave detector (probe) and converts the acoustic wave to an analog electric signal. By an electric signal processing circuit, the electric signal is amplified and AD-converted to a digital signal. Subsequently, image reconstruction is performed by a processor to generate characteristic information in each of positions in the object. The acoustic wave includes elastic wave called sound wave, ultrasound wave, or the like, and acoustic wave generated by the photoacoustic effect is particularly called photoacoustic wave or light ultrasound wave.

The characteristic information includes initial sound pressure, an optical absorption coefficient value based on the initial sound pressure, an oxygen saturation value, an optical characteristic value such as an optical energy absorption density, and further, concentration of a substance as a component of a tissue. The concentration of a substance is, for example, oxygen saturation, redox hemoglobin concentration, glucose concentration, or the like. An image expressing a characteristic distribution in an object such as an initial sound pressure distribution, an optical absorption coefficient distribution, or an oxygen saturation distribution and image data for generating an image are also obtained. Therefore, it can be said that the acoustic wave acquiring apparatus of the present invention is a photoacoustic imaging apparatus for generating an image.

In a photoacoustic imaging apparatus of the present invention, it is assumed that a light source is a pulse laser generating near-infrared light and an object is a breast of a human body or the like. Since absorption of near-infrared light by hemoglobin is high, an image can be formed from information on a spatial distribution of blood vessels in photoacoustic measurement. By visualizing information of a living organism in the object, diagnosis of a malignant tumor, a blood vessel disease, or the like of a human or animal, follow-up of chemical treatment, and the like are realized.

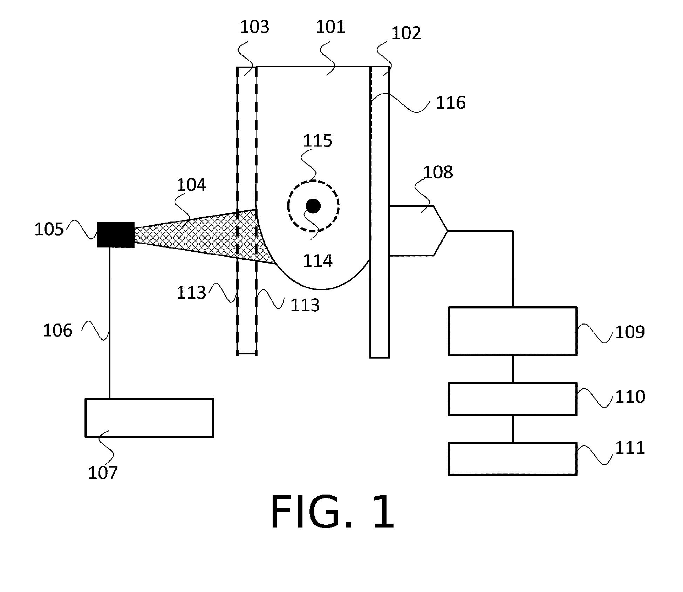

FIG. 1 is a diagram showing a basic configuration of a photoacoustic imaging apparatus according to the present invention.
A light source 107 emits light having a predetermined wavelength absorbed by a specific component (an optical absorber 114 such as hemoglobin) in components constructing a living organism. Concretely, the wavelength of light is preferably in the range of 500 nm to 1200 nm. At least one light source configured to emit pulse light of 5 nanoseconds to 50 nanoseconds is provided. A laser by which large output is obtained is preferable as the light source. In place of a laser, a light emitting diode or the like can be used. As the laser, various lasers such as a solid-state laser, a gas laser, a dye laser, and a semiconductor laser can be used. The wavelength may be tunable.

An optical irradiation unit irradiates the light emitted from the light source 107 to an object 101 and is made by an optical waveguide 106 and an irradiation optical system 105.
The optical waveguide 106 guides light emitted from the light source 107 to the irradiation optical system 105 by space propagation realized by combining optical members such as a mirror and parallel plates or propagation realized by using an optical fiber. It is sufficient to guide the light emitted from the light source 107 to the irradiation optical system 105. The irradiation optical system 105 is, for example, a member such as a mirror which reflects light, a half mirror branching light into reference light and irradiation light, a lens condensing/enlarging light and changing the shape of light, or a diffuser widening light. Any member may be used as long as it can irradiate the object 101 with the light guided by the optical waveguide in a desired shape via a holding plate 103. It is preferable to widen the irradiation light 104 to an area of a certain degree by diffusing the light by a lens. It is more preferable to irradiate the light while widening the light by the diffuser or the like. The region in which the irradiation light 104 can be applied to the object 101 is movable on the object 101. In other words, preferably, the light emitted from the light source 107 is movable on the object 101. When the irradiation region is movable, light can be irradiated in a wider range. Methods of moving the region in which the object 101 is irradiated with light include a method of using a movable mirror or the like and a method of mechanically moving the light source itself. A mechanism of moving the optical irradiation region may be integrated with the holding plate 103.

As the object 101, a target region of diagnosis such as breast, finger, hand, foot, or the like of a living body, concretely, a human body or an animal is assumed. The irradiation light 104 propagates in the object 101 and is absorbed by the optical absorber 114 and, by the photoacoustic principle, a photoacoustic wave 115 is generated and released. The optical absorber 114 has relatively high absorption coefficient in the object 101 and is, for example, a blood vessel containing much redox hemoglobin or a malignant tumor including a neovascular vessel.

An acoustic wave converting unit is made by an acoustic wave detector 108 and an electric signal processing circuit 109.
The acoustic wave detector 108 is a probe receiving, through a holding plate 102, the photoacoustic wave 115 generated from the optical absorber 114 which absorbed a part of the energy of the light propagated through the object and converting it to an electric signal. As the acoustic wave detector, any member such as a transducer using the piezoelectric phenomenon, a transducer using resonance of light, or a transducer using a change in capacitance may be used as long as it can detect an acoustic wave signal. As the transducer, a transducer in which elements are arranged in an array or a transducer made by a single element can be used.

The electric signal processing circuit 109 converts the electric signal converted by the acoustic wave detector 108 to numerical value data. That is, the acoustic wave detector performs amplifying process and AD conversion on the analog electric signal generated from the photoacoustic wave to obtain a digital electric signal, and outputs the digital electric signal.

An image reconstructing unit is made by a processor 110 and a display device 111. Any of the processor and the display device may be integrated with the part of the measuring device of the photoacoustic imaging apparatus or may be connected to the outside and operate in cooperation.

Typically, a workstation or the like is used as the processor 110. In the processor, process of calculating an initial sound pressure distribution from the numerical value data converted by the acoustic wave converting unit, calculating a light amount distribution from an optical irradiation density distribution which is preliminarily measured, and calculating an optical coefficient distribution in the object is performed by software which is programmed in advance.

Typically, a liquid crystal display or the like is used for the display device 111 on which the initial sound pressure distribution in the object, the light amount distribution, and the optical coefficient distribution are displayed.

An object holding unit sandwiches the object by two holding plates to fix the object. The object holding unit is made by the holding plates 102 and 103.

The irradiation light 104 passes through the holding plate 103 and is applied to the object 101. The photoacoustic wave 115 from the object 101 is detected by the acoustic wave detector via the holding plate 102.

The holding plate 103 on the side of irradiating the object 101 with light is a plate having high permeability to the irradiation light. For example, a transparent member to the irradiation light, for example, an organic resin such as acrylic, polycarbonate, or polymethylpentene, inorganic glass such as quartz is desired. The holding plate 103 is subjected to a waterdrop preventing process 113 as shown by broken lines.

The holding plate 102 on the side of detecting the photoacoustic wave 115 from the object 101 is a plate having high permeability to the photoacoustic wave 115. For example, acrylic, polycarbonate, polymethylpentene, or the like may be used. Particularly, polymethylpentene whose acoustic impedance is close to that of the object is preferable. Consequently, in the case of disposing the optical irradiating unit and the acoustic wave detector for the holding plate on the same side, it is preferable to use polymethylpentene.

Acoustic matching agent 116 is thinly applied to the entire contact face between the object 101 and the holding plate 102 on the side on which the acoustic wave detector 108 is disposed. The acoustic matching agent 116 is provided to enhance acoustic contact between the object 101 and the holding plate 102. As the acoustic matching agent 116, water, jel used in an ultrasonograph, or the like is desired.

Although the acoustic matching agent 116 is thinly applied between the object 101 and the acoustic wave detector 108 to enhance the acoustic contact, at the time of holding the object, it may be attached to the holding plate 103 on the side the irradiation light 104 passes. In this case, when the acoustic matching agent is attached in a waterdrop state having high contact angle, by the lens effect, the irradiation light 104 is condensed on the object 101. Conventionally, therefore, to assure safety even in the case where the irradiation light is condensed, the irradiation density of the irradiation light 104 is set sufficiently lower than that of the MPE of the JIS standard.

A concrete example of light condensation by the droplet of the acoustic matching agent will be described.

In the case of holding a breast by the holding plates with pressure, the surface of the breast is positioned in a distance of about 0 to 40 mm from the surface of the optical irradiation unit side of the holding plate 103 in accordance with the thickness of the holding plate. When the pressure plates are made of acryl and the acoustic matching agent is water, the contact angle of the waterdrop is 70°. It is assumed that the refractive index for air of water is 1.33. For example, in the case where a waterdrop having a diameter of 5 mm is adhered, by the equation (3), irradiation light having diffusion angle of $\pm 5°$ passed through the waterdrop is condensed in a position of about 8.1 mm from the waterdrop. Since the irradiation light passed through the waterdrop is irradiation light having diffusion angle of $\pm 5°$, by the equation (5) which will be described later, the light is condensed in a size having a diameter of about 1.4 mm. That is, the light having a diameter of 5 mm is condensed to about 1.4 mm. Therefore, in the case where the breast surface is positioned in distance of 8.1 mm from the waterdrop, the irradiation density of light actually emitted to the breast is about 12 times as high as that at the time of irradiation. Consequently, the density of irradiation light emitted to the object surface has to be set to about 1/12 of the MPE.

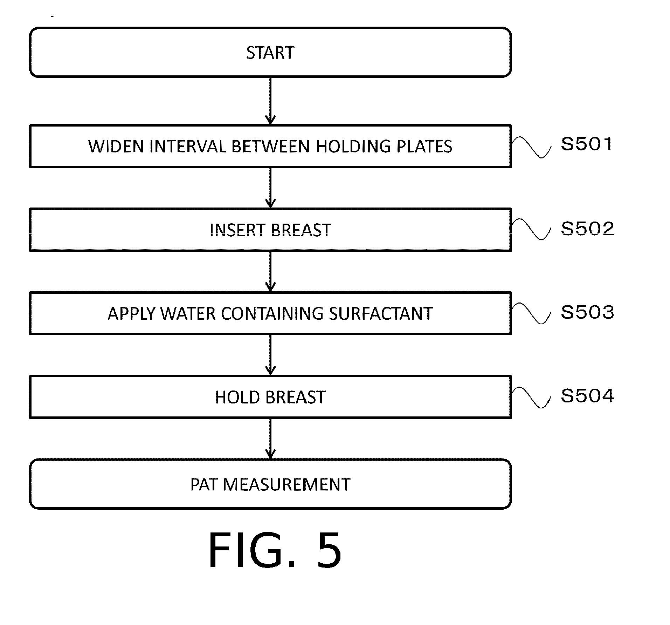

Generally, the shape of the waterdrop on a solid can be simply described as a part of a circle as shown in FIG. 5, and the contact angle $\theta$ and focal distance "f" are expressed by the equations (1) to (3). From the equations (1) to (3), it is understood that by decreasing the contact angle $\theta$, the focal distance "f" becomes longer, and the degree of focus in predetermined distance from the holding plate can be lowered.

The contact angle $\theta$ becomes about 60° to 80° in the case of acryl and water, 65° to 80° in the case of polycarbonate and water, 60° to 75° in the case of polymethylpentene and water, and about 25° in the case of quartz and water. To decrease the contact angles θ, it is considered to perform hydrophilic process on the holding plate 103. In the case of using a hydrophilic film as the hydrophilic process, the material of the film may be polyoxyethylene, polyvinyl alcohol, polyacrylic acid, or the like. Alternatively, as the hydrophilic process, soap may be coated on the holding plate 103. As hydrophilic process, a method of processing a silica coat into a fractal structure may be employed. The hydrophilic process may be performed only on the object side of the holding plate 103. However, when there is the possibility that the acoustic matching agent is adhered, the hydrophilic process may be performed on the side opposite to the object side (the optical irradiation unit side) of the holding plate 103.

By using a material containing surfactant as the acoustic matching agent, the contact angle between the acoustic matching agent and the holding plate 103 subjected to the hydrophilic process can be further decreased. As the surfactant, soap, saponin, phospholipid, peptide, or the like may be used.

When the contact angle θ becomes smaller by the hydrophilic process on the surface of the holding plate or by using the acoustic matching agent containing the surfactant, the focal distance "f" becomes longer, non-uniformity of the irradiation density distribution on the surface of the object 101 is reduced. This corresponds to enhancement of wettability to the acoustic matching agent of the holding plate.

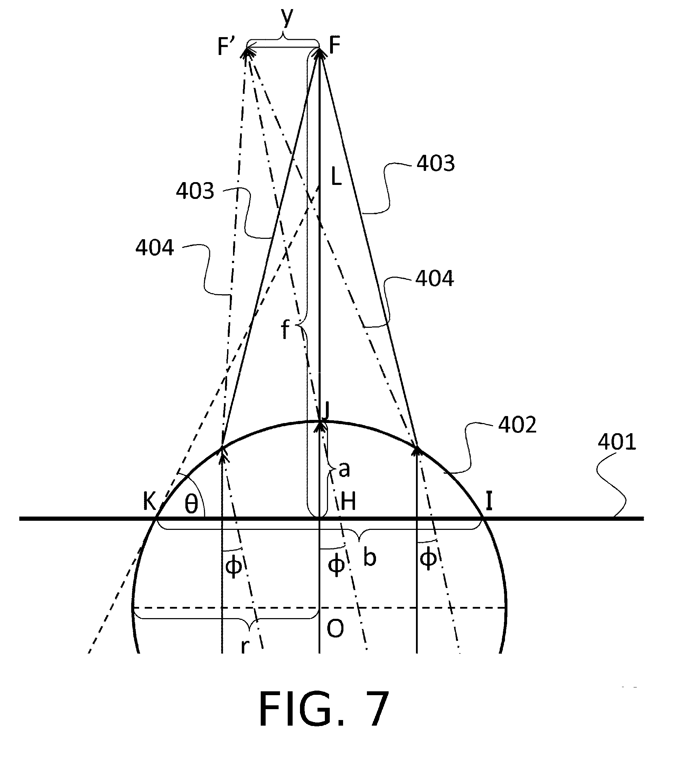

Amethod which is particularly effective to prevent the irradiation density on the surface of the subject from being locally increased will be described with reference to FIG. 7. Although laser light usually has high straightness and is hardly diffused, in this case, the laser light is intentionally diffused in the range of a predetermined angle φ or less. Irradiation light 403 which is incident perpendicular to a droplet 402 of the acoustic matching agent is focused at point F apart from the incident position by distance "f". Irradiation light 404 incident at an angle φ from the normal to the droplet is focused at point F' apart by the distance "f". When the laser light is diffused in the range of the maximum angle φ, a disc-shaped image is formed in the range of the equation (4).

$$y = f \cdot \tan\phi \qquad \ldots (4)$$

The size "y" of the image at the focal distance "f" is expressed by the equation (5) from the equations (3) and (4).

$$y \approx (b \cdot \tan\phi)/(2 \cdot (n-1) \cdot \sin\theta) \qquad \ldots (5)$$

To avoid increase in the irradiation light amount in a part of the surface of the object 101 due to focus of the irradiation light 104, it is sufficient to set the diameter of the image at the waterdrop focus position to be equal to or larger than the diameter of the waterdrop. That is, it is sufficient to satisfy the conditions of the equation (6) based on the equation (5).

$$2y/b \approx (\tan\phi)/((n-1) \cdot \sin\theta) \geq 1 \qquad \ldots (6)$$

The irradiation light 104 passes through a scatterer in the irradiation optical system 105, thereby being emitted to the object 101 with the diffusion angle φ. As the scatterer, a diffusion sheet of organic compound, diffusion glass, resin containing titanium oxide, or the like can be used.

<First Embodiment>

A photoacoustic imaging apparatus according to a first embodiment images information related to the optical absorption coefficient in the object. As the object, a breast of a human is assumed. An object holding unit is made by two parallel plates (holding plates) which hold the breast by sandwiching it.

In the embodiment, by performing the hydrophilic process on a contact part with the object, in the holding plate, the contact angle between the holding plate and the droplet of the acoustic matching agent is decreased.

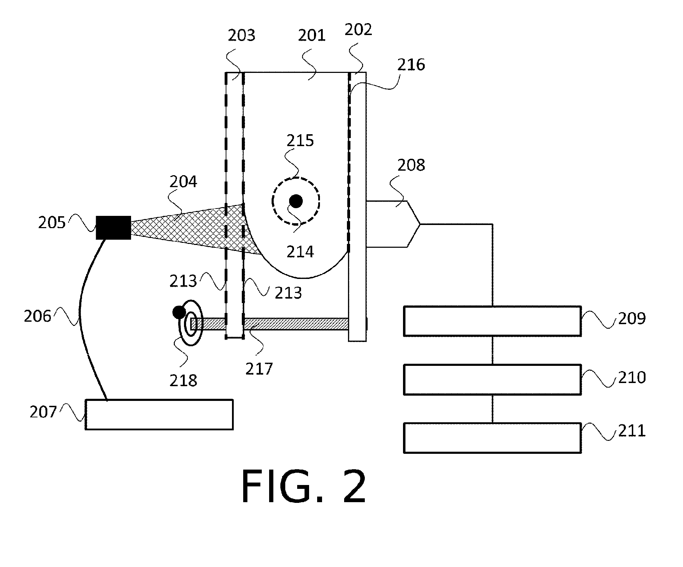

FIG. 2 shows the configuration of a main part of the photoacoustic imaging apparatus of the embodiment.

A light source 207 is a laser light source which oscillates light having a wavelength 797 nm and is a Ti:sa laser using an Nd:YAG laser having a pulse width 10 nsec and a recurrence frequency 10 Hz as an excitation light source. An optical waveguide 206 is made by an optical fiber and transmits laser light emitted from the light source 207 to an irradiation optical system 205. The irradiation optical system 205 emits irradiation light 204 having diffusion of ±5° through a lens diffuser which enlarges the light transmitted by the optical waveguide 206 and diffuses at ±5° to a breast 201 via a movable holding plate 203. The object of the embodiment is a breast.

An acoustic wave detector 208 is a two-dimensional array piezoelectric probe, is opposed to the irradiation light 204 over the object, and is in contact with a fixed holding plate 202. The acoustic wave detector 208 and the fixed holding plate 202 are acoustically matched by acoustic matching agent (not shown). The acoustic wave detector 208 receives a photoacoustic wave 215 generated when an optical absorber 214 in the breast 201 is irradiated with light. In the embodiment, the optical absorber is a neovascular vessel in a cancer in the breast.

The acoustic wave detector 208 converts the receivedphotoacoustic wave to an analog electric signal. An electric signal processing circuit 209 performs processes such as amplification and AD conversion on an analog electric signal. A processor in the embodiment is a workstation 210. The workstation calculates an optical coefficient distribution in the breast 201 by using the data subjected to the electric signal process. The optical coefficient distribution is displayed on a liquid crystal display 211 as the display device of the embodiment.

An object holding unit is made by the fixed holding plate 202, the movable holding plate 203, and a holding width changing jig 217. The fixed holding plate 202 is made of polymethylpentene, and the movable holding plate 203 is an acryl plate. Light is emitted from the side of the movable holding plate 203 to the object, and photoacoustic wave is received by an acoustic wave detector disposed on the side of the fixed holding plate 202. The fixed holding plate 202 and the movable holding plate 203 are arranged almost in parallel and are almost perpendicular to the floor face. The user operates a holding width changing handle 218 to move the movable holding plate 203 through the holding width changing jig 217, so that the distance to the fixed holding plate 202 can be changed. The breast 201 is held so as to be sandwiched by the fixed holding plate 202 and the movable holding plate 203. A hydrophilic process 213 is performed on the movable holding plate 203. The hydrophilic process in the embodiment is soap coating.

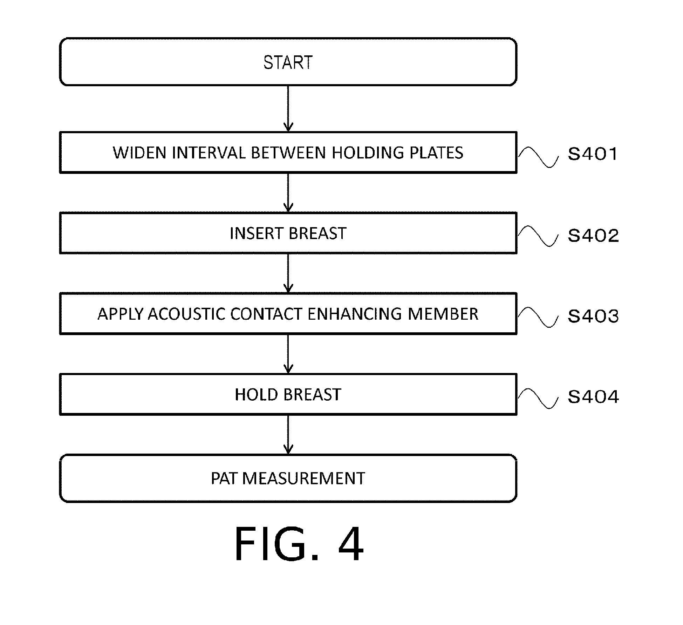

The flowchart of FIG. 4 shows a method of holding the breast 201.

In step S401, the interval between the fixed holding plate 202 and the movable holding plate 203 is widened by the holding width changing handle 218.

In step S402, the breast 201 is inserted in the widened space.

In step S403, water is applied as acoustic matching agent 216 between the breast 201 and the fixed holding plate 202.

In step S404, the interval between the fixed holding plate 202 and the movable holding plate 203 is narrowed to hold the breast 201. The breast 201 is held and PAT measurement can be performed.

When water is applied as the acoustic matching agent between the breast 201 and the fixed holding plate 202 as in the above procedure, at the time of holding the breast 201, there is the possibility that the water is adhered not only to the fixed holding plate 202 but also to the movable holding plate 203. As a result, there is the possibility that a waterdrop is interposed in a space until the irradiation light 204 emitted from the irradiation optical system 205 reaches the surface of the breast 201 and light is focused.

In the case of holding the breast 201 with pressure, in the mechanism of the embodiment, the distance between the fixed holding plate 202 and the movable holding plate 203 is 40 to 60 mm. The irradiated face of the breast 201 has a part which is in contact with the movable holding plate 203 and a part which is apart from the movable holding plate 203. In this case, the distance between the irradiated face and the movable holding plate is in the range of 0 to 40 mm.

Focus in the case where a drop of the acoustic matching agent is adhered to the movable holding plate 203 will be examined. In the case where the hydrophilic process is not performed on the movable holding plate 203, since the movable holding plate 203 is made of acrylic and the acoustic matching agent is water, the contact angle is 70°. For example, when it is assumed that the diameter of the waterdrop is 5 mm, the diffusion angle of the irradiation light is $\pm 5°$, and the refractive index for air of water is 1.33, as described above using the computation expression, the irradiation light 204 passed through the waterdrop is focused in the size of the diameter of about 1.4 mm in the position of about 8.1 mm from the waterdrop. That is, the light having the diameter 5 mm becomes about 1.4 mm. Therefore, when the hydrophilic process is not performed, in the case where the breast surface is positioned apart from the waterdrop by 8.1 mm, the irradiation density becomes about 12 times.

On the other hand, in the present embodiment, the movable holding plate 203 is coated with soap as the hydrophilic process, so that the contact angle of the waterdrop decreases to 10° or less. In the case where a waterdrop having a diameter of 5 mm is adhered, since the refractive index for air of water is 1.33, the irradiation light 204 passed through the waterdrop has a focal position apart from the waterdrop by about 8.1 mm. Since the irradiation light is light having the diffusion angle of $\pm 5°$, the irradiation light 204 passed through the waterdrop comes to have the diameter of about 7.6 mm by the equation (5). That is, even if a waterdrop is adhered to the movable holding plate subjected to the hydrophilic process as in the embodiment, focusing does not happen.

By performing the hydrophilic process on the surface of the holding plate so that the contact angle becomes small (for example, 10° or less), focusing in the surface of the breast is improved. As a result, it becomes unnecessary to excessively decrease the intensity of the irradiation light, and an image having a preferable S/N ratio can be constructed.

<Second Embodiment>

A photoacoustic imaging apparatus according to a second embodiment is **characterized in that** the contact angle between a pressing plate and acoustic matching agent is decreased by using acoustic matching agent containing a surfactant.

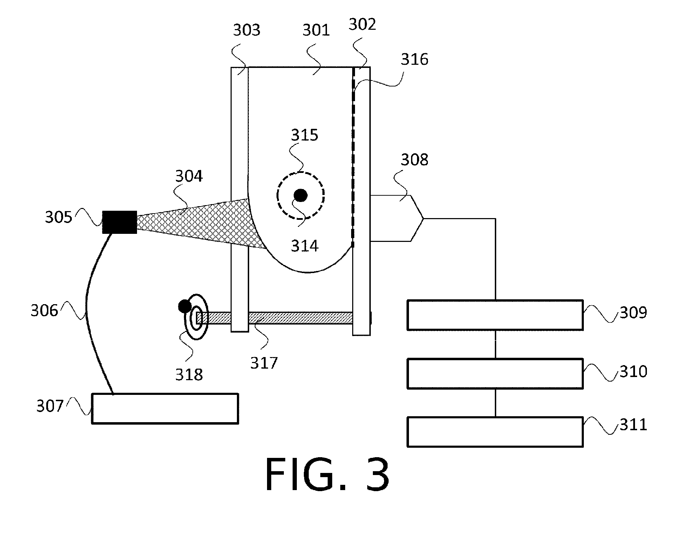

FIG. 3 shows the configuration of a main part of the photoacoustic imaging apparatus of the embodiment. Since the configuration of the apparatus is similar to that described in the first embodiment, characteristic parts of the second embodiment will be described.

In the second embodiment, an irradiation optical system 305 enlarges light transmitted through an optical waveguide 306 to obtain irradiation light 304 having diffusion of $\pm 5°$ through a resin (scattering medium) containing a scatterer which diffuses light to $\pm 5°$.

In the embodiment, a movable holding plate 303 is made of quartz glass. In the embodiment, although it is not shown since the hydrophilic process is not always necessary for the movable holding plate, various hydrophilic processes 313 may be used concurrently.

As a characteristic of the embodiment, water containing surfactant is used as acoustic matching agent 316 applied between a breast 301 and a fixed holding plate 302.

The flowchart of FIG. 5 shows a method of holding the breast 301.

In step S501, the interval between the fixed holding plate 302 and the movable holding plate 303 is widened by a holding width changing handle 318.

In step S502, the breast 301 is inserted in the widened space.

In step S503, water containing surfactant is applied between the breast 301 and the fixed holding plate 302.

In step S504, the interval between the fixed holding plate 302 and the movable holding plate 303 is narrowed to hold the breast 301. The breast 301 is held and PAT measurement can be performed.

In step S503, as the water containing surfactant, soapy water is used. As a result, the contact angle of the soapy water on the quartz glass becomes 15° or less. With saponin, phospholipid, peptide, or the like as the surfactant, a similar effect is obtained.

In a manner similar to the first embodiment, the distance between the fixed holding plate 302 and the movable holding plate 303 in the case of holding the breast 301 with pressure becomes 40 mm to 60 mm. The distance between the irradiated face of the breast 301 and the movable holding plate is in the range of 0 to 40 mm.

Focus in the case where a drop of the acoustic matching agent is adhered to the movable holding plate 303 will be examined. In the case where the hydrophilic process is not performed on the movable holding plate 303 and the acoustic matching agent does not contain surfactant, since the movable holding plate 303 is made of quartz and the acoustic matching agent is water, the contact angle is 25°. For example, when it is assumed that the diameter of the waterdrop is 5 mm, the diffusion angle of the irradiation light is $\pm 5°$, and the refractive index for air of water is 1.33, it is understood that the irradiation light 304 passed through the waterdrop is focused in the size of the diameter of about 3.1 mm in the position of about 17.9 mm from the waterdrop by the equations (3) and (5). That is, the light having the diameter 5 mm becomes about 3.1 mm. Therefore, when the surfactant is not used, in the case where the breast surface is positioned apart from the waterdrop by 17.9 mm, the irradiation density becomes about 2.6 times.

On the other hand, in the present embodiment, since the water containing the surfactant is used as the acoustic matching agent, the contact angle of the waterdrop adhered to the movable holding plate 303 made of quartz glass decreases to 10° or less. In the case where a waterdrop having a diameter of 5 mm is adhered, since the refractive index for air of water is 1.33, the irradiation light 304 passed through the waterdrop has a focal position apart from the waterdrop by about 29.3 mm from the equation (3). Since the irradiation light is light having the diffusion angle of $\pm 5°$, the irradiation light 304 passed through the waterdrop comes to have the diameter of about 5.1 mm by the equation (5). That is, even if a waterdrop of the acoustic matching agent containing the surfactant is adhered to the movable holding plate like in the embodiment, focusing does not happen.

The water containing the surfactant spreads in a film state. For example, when it is assumed that a waterdrop containing the surfactant and having a contact angle of 10° and a diameter of 5 mm is adhered to the movable holding plate made of quartz glass, the focal distance is obtained from the equation (2) as 40 mm or longer (43.2 mm). Consequently, light is not focused also on the irradiated face of the breast 301, and the irradiation density distribution does not become non-uniform.

Further, it is assumed that the diffusion angle of the irradiation light 304 passed through the diffuser in the irradiation optical system 305 becomes $\pm 5°$. In this case, in the equation (6), the left side becomes 1.52, and the condition of an inequality is satisfied. That is, light passed through a waterdrop having a diameter of 5 mm is not focused in a focal position (43.2 mm from the waterdrop), and the optical irradiation density distribution does not become non-uniform.

In the embodiment, by using the water containing the surfactant as the acoustic matching agent, the contact angle becomes small (for example, 15° or less). As a result, focusing of the irradiation density distribution of the breast surface is improved, so that light having higher density can be emitted, and an image having a preferable S/N ratio can be generated.

In each of the foregoing embodiments, the example where the irradiation optical system and the acoustic wave detector are disposed for different holding plates has been described on precondition of the mechanism of sandwiching a breast as an object by using two holding plates and holding the breast with pressure. The present invention can be applied to a photoacoustic imaging apparatus (acoustic wave acquiring apparatus) in which a waterdrop may be adhered to a holding plate and light may be focused. For example, the present invention can be also applied to the case where the irradiation optical system and the acoustic wave detector are disposed for the same holding plate or the case where both faces of a configuration using two holding plates are irradiated with light. That is, by performing the hydrophilic process on the surface of a holding plate or by making the surfactant contained in the acoustic matching agent, the contact angle between the waterdrop and the holding plate is decreased, and excessive focusing can be prevented.

While the present invention has been described with reference to exemplary embodiments, it is to be understood that the invention is not limited to the disclosed exemplary embodiments. The scope of the following claims is to be accorded the broadest interpretation so as to encompass all suchmodifications and equivalent structures and functions. The present invention provides an acoustic wave acquiring apparatus including: a holding plate configured to hold an object via acoustic matching agent; an acoustic wave detector configured to receive an acoustic wave generated from the object irradiated with light from a light source and convert the acoustic wave to an electric signal; and a processor configured to generate characteristic information of the inside of the object by using the electric signal. In the acoustic wave acquiring apparatus, the holding plate is subjected to hydrophilic process or the acoustic matching agent contains surfactant.

**Claims**

1.  An acoustic wave acquiring apparatus comprising:

    a holding plate configured to hold an object via acoustic matching agent;
    an acoustic wave detector configured to receive an acoustic wave generated from the object irradiated with light from a light source and convert the acoustic wave to an electric signal; and
    a processor configured to generate characteristic information of the inside of the object by using the electric signal, wherein the holding plate is subjected to hydrophilic process.

2.  The acoustic wave acquiring apparatus according to claim 1, wherein the hydrophilic process is soap coating on the holding plate.

3.  An acoustic wave acquiring apparatus comprising:

    a holding plate configured to hold an object via acoustic matching agent;
    an acoustic wave detector configured to receive an acoustic wave generated from the object irradiated with light from a light source and convert the acoustic wave to an electric signal; and
    a processor configured to generate characteristic information of the inside of the object by using the electric signal, wherein the acoustic matching agent contains surfactant.

4.  The acoustic wave acquiring apparatus according to claim 3, wherein the surfactant is soapy water.

5.  The acoustic wave acquiring apparatus according to any one of claims 1 to 4, further comprising an irradiation optical system configured to diffuse light from the light source and irradiate the object with the light.

6.  The acoustic wave acquiring apparatus according to claim 5, wherein when a contact angle between the holding plate and the acoustic matching agent is $\theta$, diffusion angle of light from the irradiation optical system is $\phi$, and refractive index for air of the acoustic matching agent is n, the following equation is satisfied.

$$(\tan\phi)/((n-1)\cdot\sin\theta) \geq 1$$

FIG. 1

FIG. 2

# FIG. 3

```
┌─────────────────────────────────────────────────┐
│                      START                       │
└─────────────────────────────────────────────────┘
                        │
                        ▼
┌─────────────────────────────────────────────────┐
│      WIDEN INTERVAL BETWEEN HOLDING PLATES       │  ∿  S401
└─────────────────────────────────────────────────┘
                        │
                        ▼
┌─────────────────────────────────────────────────┐
│                  INSERT BREAST                   │  ∿  S402
└─────────────────────────────────────────────────┘
                        │
                        ▼
┌─────────────────────────────────────────────────┐
│      APPLY ACOUSTIC CONTACT ENHANCING MEMBER     │  ∿  S403
└─────────────────────────────────────────────────┘
                        │
                        ▼
┌─────────────────────────────────────────────────┐
│                   HOLD BREAST                    │  ∿  S404
└─────────────────────────────────────────────────┘
                        │
                        ▼
┌─────────────────────────────────────────────────┐
│                 PAT MEASUREMENT                  │
└─────────────────────────────────────────────────┘
```

# FIG. 4

```
┌─────────────────────────────────────────┐
│                 START                     │
└─────────────────────────────────────────┘
                    │
                    ▼
┌─────────────────────────────────────────┐
│  WIDEN INTERVAL BETWEEN HOLDING PLATES    │ ∿ S501
└─────────────────────────────────────────┘
                    │
                    ▼
┌─────────────────────────────────────────┐
│              INSERT BREAST                │ ∿ S502
└─────────────────────────────────────────┘
                    │
                    ▼
┌─────────────────────────────────────────┐
│     APPLY WATER CONTAINING SURFACTANT     │ ∿ S503
└─────────────────────────────────────────┘
                    │
                    ▼
┌─────────────────────────────────────────┐
│               HOLD BREAST                 │ ∿ S504
└─────────────────────────────────────────┘
                    │
                    ▼
┌─────────────────────────────────────────┐
│             PAT MEASUREMENT               │
└─────────────────────────────────────────┘
```

# FIG. 5

FIG. 6

FIG. 7

Europäisches
Patentamt

European
Patent Office

Office européen
des brevets

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>A | WO 2010/084990 A1 (CANON KK [JP]; TOKITA TOSHINOBU [JP]) 29 July 2010 (2010-07-29)<br>* abstract *<br>* page 10, line 25 - page 18, line 17 *<br>* claims 7-9; figures 1,2 *<br>----- | 1,2,5<br><br>3,4 | INV.<br>A61B8/08<br>A61B5/00 |
| A | WO 2010/030817 A1 (ENDRA INC [US]; THORNTON MICHAEL M [CA]; KRUGER ROBERT A [US]) 18 March 2010 (2010-03-18)<br>* the whole document *<br>----- | 1,3,5 | |
| A | US 2006/094944 A1 (CHUANG HAN [US])<br>4 May 2006 (2006-05-04)<br>* abstract *<br>* paragraph [0080] *<br>* paragraph [0155] - paragraph [0157] *<br>----- | 1,3,4 | |
| A,D | US 5 713 356 A (KRUGER ROBERT A [US])<br>3 February 1998 (1998-02-03)<br>* the whole document *<br>----- | 1,3,5 | |

| TECHNICAL FIELDS SEARCHED (IPC) |
|---|
| A61B |

The present search report has been drawn up for all claims

| Place of search | Date of completion of the search | Examiner |
|---|---|---|
| Munich | 14 January 2013 | Artikis, T |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 2 578 164 A1**

## ANNEX TO THE EUROPEAN SEARCH REPORT
## ON EUROPEAN PATENT APPLICATION NO.

EP 12 18 5176

This annex lists the patent family members relating to the patent documents cited in the above-mentioned European search report.
The members are as contained in the European Patent Office EDP file on
The European Patent Office is in no way liable for these particulars which are merely given for the purpose of information.

14-01-2013

| Patent document cited in search report | | | Publication date | Patent family member(s) | | | Publication date |
|---|---|---|---|---|---|---|---|
| WO 2010084990 | A1 | | 29-07-2010 | JP | 2010167001 | A | 05-08-2010 |
| | | | | US | 2011245667 | A1 | 06-10-2011 |
| | | | | WO | 2010084990 | A1 | 29-07-2010 |
| WO 2010030817 | A1 | | 18-03-2010 | CA | 2736868 | A1 | 18-03-2010 |
| | | | | CN | 102223840 | A | 19-10-2011 |
| | | | | EP | 2352422 | A1 | 10-08-2011 |
| | | | | US | 2011306865 | A1 | 15-12-2011 |
| | | | | WO | 2010030817 | A1 | 18-03-2010 |
| US 2006094944 | A1 | | 04-05-2006 | BR | PI0517255 | A | 07-10-2008 |
| | | | | NZ | 554624 | A | 26-11-2010 |
| | | | | US | 2006094944 | A1 | 04-05-2006 |
| | | | | US | 2006094945 | A1 | 04-05-2006 |
| | | | | US | 2006094946 | A1 | 04-05-2006 |
| | | | | US | 2008311670 | A1 | 18-12-2008 |
| US 5713356 | A | | 03-02-1998 | AT | 408374 | T | 15-10-2008 |
| | | | | AU | 725072 | B2 | 05-10-2000 |
| | | | | BR | 9712262 | A | 25-01-2000 |
| | | | | CA | 2187701 | A1 | 04-04-1998 |
| | | | | EP | 0942683 | A1 | 22-09-1999 |
| | | | | JP | 4341987 | B2 | 14-10-2009 |
| | | | | JP | 2001507952 | A | 19-06-2001 |
| | | | | US | 5713356 | A | 03-02-1998 |
| | | | | US | 6102857 | A | 15-08-2000 |
| | | | | US | 6292682 | B1 | 18-09-2001 |
| | | | | US | 2002035327 | A1 | 21-03-2002 |
| | | | | WO | 9814118 | A1 | 09-04-1998 |

EPO FORM P0459

For more details about this annex : see Official Journal of the European Patent Office, No. 12/82

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

•   US 5713356 A

**Non-patent literature cited in the description**

•   **SRIRANG MANOHAR et al.** Characterization of a clinical prototype for photoacoustic mammographu and some phantom studies. *Proc. Of SPIE,* 27